# EUROPEAN PATENT APPLICATION

(11) **EP 4 785 854 A1**
(43) Date of publication of application: **05.08.2026**
(21) Application number: 24872810.7
(22) Date of filing: 13.09.2024
(51) Int. Cl.: A61B 5/00, G16H 50/20, G06N 3/08

(54) **DEVICE, METHOD, AND USER INTERFACE FOR DETERMINING BIO-SIGNAL MEASUREMENT CYCLE**

(30) Priority: 26.09.2023 KR 20230128990
(71) Applicant: VUNO Inc., Seoul 06541 (KR)
(72) Inventor: CHANG, Mineok, Seoul 06655 (KR); JOO, Sunghoon, Seoul 04778 (KR)
(74) Representative: Buzzi, Notaro & Antonielli d'Oulx S.p.A.
(86) International application number: PCT/KR2024/013964
(87) International publication number: WO 2025/071102

(57) **Abstract**

According to an embodiment of the present disclosure, a method for determining a bio signal measurement interval is disclosed. Specifically, according to the present disclosure, a computing device receives a bio signal of a user, and determines a personalized bio signal measurement interval of the user based on inputting the bio signal of the user into a bio signal analysis model, wherein the bio signal analysis model comprises at least one bio signal analysis sub-model that analyzes a health condition of the user.

## Description

### [Technical Field]

The present disclosure relates to a method for determining measurement interval of bio signal, and more specifically, to a method for determining a personalized bio signal measurement interval of a user by inputting a bio signal of the user into a bio signal analysis model comprising at least one bio signal analysis sub-model.

### [Background Art]

Recently, as services and additional functions provided by a personal portable device such as a smart phone have diversified, various applications meeting demands of consumers have been developed.

Meanwhile, in a mobile health care field, a health management system in which a bio signal including an electrocardiogram (ECG) of a user is measured using an instrument or the like, and then transmitted to a portable device such as a smart phone or a tablet PC so that the device provides various diagnostic information and the like is in the spotlight.

In order to effectively operate such a health management system, it is necessary to periodically measure the bio signal of the user to continuously track changes in a health condition. For periodic measurement, an alarm requesting measurement of the bio signal may be transmitted to the user at a predetermined interval, but when such an alarm is repeated at the predetermined interval, alarm fatigue may occur. When the above situation occurs, there is a possibility that the user rather turns off the alarm, and thus, there occurs a problem that it becomes impossible to track the changes in the health condition. In particular, in the case of a user who needs to measure the bio signal more frequently because a large change in the bio signal is observed, such a problem may cause a health risk.

Therefore, there is a demand in the art for a method of determining a bio signal measurement interval optimized for each user and transmitting an alarm requesting the measurement.

Korean Patent Registration No. 10-2525996 discloses an apparatus for measuring a bio signal.

### [Disclosure]

### [Technical Problem]

The present disclosure is designed in response to the above-described background art, and an object of the present disclosure is to provide a method of determining a personalized bio signal measurement interval of a user by inputting a bio signal of the user into a bio signal analysis model comprising at least one bio signal analysis sub-model, and to provide an alarm requesting bio signal measurement in the form of a user interface.

Meanwhile, the technical problems to be solved by the present disclosure are not limited to the above-mentioned technical problems, and various technical problems may be included within a range apparent to those skilled in the art from the contents to be described below.

### [Technical Solution]

According to an embodiment of the present disclosure for realizing the above-described problems, a method performed by a computing device for determining a bio signal measurement interval is disclosed. The method is a method performed by a computing device for determining a bio signal measurement interval, the method comprising: receiving a bio signal of a user; and determining a personalized bio signal measurement interval of the user based on inputting the bio signal of the user into a bio signal analysis model, wherein the bio signal analysis model may comprise at least one bio signal analysis sub-model that analyzes a health condition of the user.

In an embodiment, determining the personalized bio signal measurement interval may comprise: inputting at least a part of the bio signal of the user into the at least one bio signal analysis sub-model; generating comprehensive diagnosis information on the health condition of the user based on an output value generated by the at least one bio signal analysis sub-model; and determining the personalized bio signal measurement interval based on the comprehensive diagnosis information.

In an embodiment, at least a part of the at least one bio signal analysis sub-model may comprise an artificial neural network model, and the comprehensive diagnosis information may be obtained based on disease diagnosis information output by the artificial neural network model.

In an embodiment, the method may further comprise: displaying a user interface related to the personalized bio signal measurement interval, wherein the user interface may comprise an area indicating whether the personalized bio signal measurement interval is changed, and an area displaying the personalized bio signal measurement interval.

In an embodiment, the method may further comprise: displaying a user interface related to a change in the personalized bio signal measurement interval in response to a selection input of the user, wherein the user interface related to the change in the personalized bio signal measurement interval may comprise an area displaying details of the change in the personalized bio signal measurement interval, and an area displaying at least one reason for change in the personalized bio signal measurement interval.

In an embodiment, the method may further comprise: displaying a user interface related to the selected reason for change in response to receiving a selection input related to a reason for change in the bio signal measurement interval of the user, wherein the user interface related to the selected reason for change may comprise an area displaying information related to a measurement time point of the bio signal, and an area displaying disease diagnosis information according to the measurement time.

In an embodiment, the method may further comprise: generating alarm information requesting the user to measure the bio signal, based on the personalized bio signal measurement interval.

In an embodiment, generating the alarm information may comprise: displaying a user interface related to the alarm information, wherein the user interface related to the alarm information may comprise an area displaying information related to a request for a bio signal measurement; an area displaying information related to a past bio signal measurement result; an area displaying information related to the personalized bio signal measurement interval; and an area displaying information related to a reason for change in the personalized bio signal measurement interval.

According to an embodiment of the present disclosure for realizing the above-described problems, a computer program stored in a computer-readable storage medium causing a computing device to perform operations for determining a bio signal measurement interval is disclosed. The operations comprise: receiving a bio signal of a user; and determining a personalized bio signal measurement interval of the user based on inputting the bio signal of the user into a bio signal analysis model, wherein the bio signal analysis model may comprise at least one bio signal analysis sub-model that analyzes a health condition of the user.

According to an embodiment of the present disclosure for realizing the above-described problems, a computing device for determining a bio signal measurement interval is disclosed. The computing device comprises at least one processor and a memory, wherein the at least one processor is configured to receive a bio signal of a user; and determine a personalized bio signal measurement interval of the user based on inputting the bio signal of the user into a bio signal analysis model, wherein the bio signal analysis model may comprise at least one bio signal analysis sub-model that analyzes a health condition of the user.

### [Advantageous Effects]

According to the present disclosure, by analyzing the bio signal of the user and detecting an abnormality of the bio signal, it is possible to determine an optimized bio signal measurement interval for a user who needs continuous monitoring due to an increased risk of a disease. In addition, by providing a change in the bio signal measurement interval, a reason for change, and an alarm requesting measurement of the bio signal in the form of a user interface, it is possible to increase compliance of the user with the alarm. Therefore, according to the present disclosure, an effect of increasing satisfaction of the user with the use of a service occurs in operating a health management system utilizing a portable device.

### [Description of Drawings]

Figure 1 is a block diagram of a computing device for determining a bio signal measurement interval according to an embodiment of the present disclosure.
Figure 2 is a schematic diagram illustrating a network function according to an embodiment of the present disclosure.
Figure 3 is a flowchart illustrating a process of determining a personalized bio signal measurement interval of a user according to an embodiment of the present disclosure.
Figure 4a is a conceptual diagram illustrating a user interface related to a personalized bio signal measurement interval according to an embodiment of the present disclosure.
Figure 4b is a conceptual diagram illustrating a user interface related to a change in a personalized bio signal measurement interval according to an embodiment of the present disclosure.
Figure 4c is a conceptual diagram illustrating a user interface related to a reason for change in a personalized bio signal measurement interval according to an embodiment of the present disclosure.
Figure 5 is a conceptual diagram illustrating a user interface related to an alarm requesting a bio signal measurement according to an embodiment of the present disclosure.
Figure 6 is a brief and general schematic diagram of an exemplary computing environment in which embodiments of the present disclosure may be implemented.

### [Best Mode]

The present disclosure discloses a method for determining a personalized bio signal measurement interval of a user by inputting a bio signal of the user into a bio signal analysis model comprising at least one bio signal analysis sub-model, and a method of providing an alarm requesting bio signal measurement in the form of a user interface.

Various embodiments are now described with reference to the drawings. In the present specification, various descriptions are presented to provide an understanding of the present disclosure. However, it is apparent that these embodiments may be practiced without these specific descriptions.

As used herein, the terms "component", "module", "system", and the like refer to a computer-related entity, hardware, firmware, software, a combination of software and hardware, or execution of software. For example, a component may be, but is not limited to, a procedure executed on a processor, a processor, an object, a thread of execution, a program, and/or a computer. For example, both an application executing on a computing device and the computing device may be components. One or more components may reside within a processor and/or thread of execution. A component may be localized on one computer. In the present disclosure, one or more components may be distributed between two or more computers, i.e., execution environments. In addition, these components may execute from various computer-readable media having various data structures stored thereon. Components may communicate via local and/or remote processes, such as in accordance with a signal having one or more data packets (e.g., data from one component interacting with another component in a local system, a distributed system, and/or across a network such as the Internet via the signal).

In addition, the term "or" is intended to mean an inclusive "or" rather than an exclusive "or". That is, unless otherwise specified or clear from context, "X employs A or B" is intended to mean any of the natural inclusive permutations. That is, if X employs A; X employs B; or X employs both A and B, then "X employs A or B" is satisfied under any of the foregoing instances. Also, the term "and/or" as used herein should be understood to refer to and encompass any and all possible combinations of one or more of the associated listed items.

Furthermore, the terms "comprises" and/or "comprising" should be understood to mean that the corresponding features and/or components are present. However, the terms "comprises" and/or "comprising" should be understood as not excluding the presence or addition of one or more other features, components, and/or groups thereof. Also, unless specified otherwise or clear from context to be directed to a singular form, the singular forms in the present specification and claims should generally be construed to mean "one or more".

And, the term "at least one of A or B" should be interpreted to mean "a case including only A", "a case including only B", and "a case in which A and B are combined".

Those skilled in the art should further appreciate that the various illustrative logical blocks, configurations, modules, circuits, means, logics, and algorithm steps described in connection with the embodiments disclosed herein may be implemented as electronic hardware, computer software, or combinations of both. To clearly illustrate this interchangeability of hardware and software, various illustrative components, blocks, configurations, means, logics, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application. However, such implementation decisions should not be interpreted as causing a departure from the scope of the present disclosure.

The description of the presented embodiments is provided to enable any person skilled in the art to make or use the present disclosure. Various modifications to these embodiments will be readily apparent to those skilled in the art. The generic principles defined herein may be applied to other embodiments without departing from the scope of the present disclosure. Thus, the present disclosure is not intended to be limited to the embodiments shown herein. The present disclosure is to be accorded the widest scope consistent with the principles and novel features presented herein.

In the present disclosure, the bio signal may include a vital sign. Generally, the bio signal may refer to an electrical signal or a non-electrical signal that can be continuously measured from a user (subject). The bio signal may include various biorelated signals such as an electrocardiogram (ECG), an electromyogram (EMG), an electroencephalogram (EEG), electrodermal activity (EDA), a skin temperature (SKT), a photoplethysmography (PPG), a vital sign, an X-ray image signal, a magnetic resonance imaging (MRI) signal, a computed tomography (CT) image signal, and the like. Furthermore, the bio signal is not limited to these examples, and may include various information obtained from the user using a medical device, a wearable device, a mobile device, and the like.

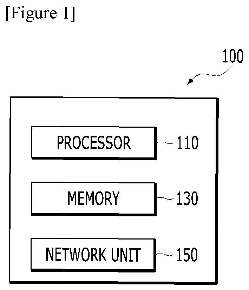

Figure 1 is a block diagram of a computing device for determining a bio signal measurement interval according to an embodiment of the present disclosure.

The configuration of the computing device 100 shown in Figure 1 is only an example shown in a simplified manner. In an embodiment of the present disclosure, the computing device 100 may include other components for executing a computing environment of the computing device 100, and only some of the disclosed components may configure the computing device 100.

The computing device 100 may include a processor 110, a memory 130, and a network unit 150.

The processor 110 may be configured with one or more cores, and may include processors for data analysis and deep learning, such as a central processing unit (CPU), a general purpose graphics processing unit (GPGPU), and a tensor processing unit (TPU) of the computing device. The processor 110 may read a computer program stored in the memory 130 to perform data processing for machine learning according to an embodiment of the present disclosure. According to an embodiment of the present disclosure, the processor 110 may perform calculations for learning a neural network. The processor 110 may perform calculations for learning the neural network in deep learning (DL), such as processing input data for learning, extracting features from the input data, calculating an error, and updating weights of the neural network using backpropagation.

At least one of the CPU, GPGPU, and TPU of the processor 110 may process learning of a network function. For example, the CPU and GPGPU may process learning of a network function and data classification using the network function together. In addition, in an embodiment of the present disclosure, processors of a plurality of computing devices may be used together to process learning of a network function and data classification using the network function. Also, a computer program executed in a computing device according to an embodiment of the present disclosure may be an executable program for a CPU, GPGPU, or TPU.

In an embodiment of the present disclosure, the processor 110 may receive a bio signal of a user. In the present disclosure, the bio signal may be data measured from a separate bio signal measurement device (not shown) connected to the computing device 100 and received through the network unit 150, but the computing device 100 may include an input unit (not shown) for measuring the bio signal, and in this case, the processor 110 may receive the bio signal measured by the input unit (not shown). In addition to the above examples, the processor 110 may receive the bio signal of the user stored in the memory 130, and the method of receiving the bio signal in the present disclosure is not limited to the above examples.

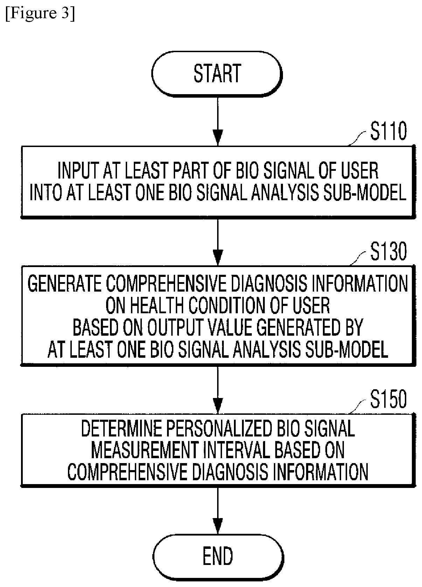

The processor 110 may determine a personalized bio signal measurement interval of the user based on inputting the bio signal of the user into a bio signal analysis model. In the present disclosure, the bio signal analysis model may include at least one bio signal analysis sub-model, and the at least one bio signal analysis sub-model may include an artificial neural network model. For example, the bio signal analysis model of the present disclosure may include at least one or more of a blood pressure analysis sub-model, a blood sugar analysis sub-model, or an electrocardiogram (ECG) analysis sub-model. A specific method of determining a personalized bio signal measurement interval of a user by utilizing a bio signal analysis model will be described later with reference to Figure 3.

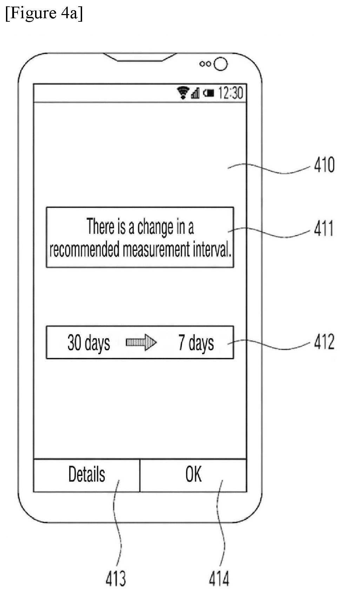

The processor 110 may display a user interface related to the personalized bio signal measurement interval through a touch screen (not shown) or the like. Specific examples of the user interface related to the personalized bio signal measurement interval will be described later with reference to FIGS. 4a to 4c.

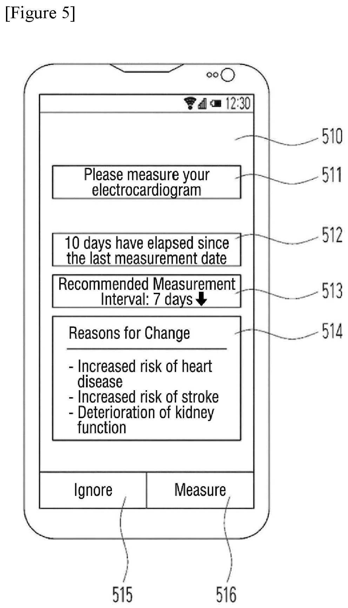

The processor 110 may generate alarm information requesting the user to measure a bio signal based on the personalized bio signal measurement interval, and may display a user interface related to the alarm on a touch screen (not shown) or the like. A specific example of the user interface related to the alarm will be described later with reference to Figure 5.

Through the present disclosure, by analyzing the bio signal of the user and detecting an abnormality of the bio signal, it is possible to determine an optimized bio signal measurement interval for a user who needs continuous monitoring due to an increased risk of a disease, for example, a user for whom an estimated heart age at a current time and an actual age have a difference equal to or greater than a threshold value, a user for whom a predicted heart age change at a plurality of future times is equal to or greater than a threshold value, and a user for whom a change between a heart age estimated at a past time and a heart age estimated at a current time is equal to or greater than a threshold value, by estimating the heart age at the current time or predicting heart ages at the plurality of future times upon receiving ECG data. Alternatively, an optimized bio signal measurement interval may be determined for a user whose kidney function has deteriorated by receiving ECG data and estimating a serum potassium level and an estimated glomerular filtration rate (eGFR). In addition, by providing a change in the bio signal measurement interval, a reason for change, and an alarm requesting measurement of the bio signal in the form of a user interface, it is possible to increase compliance of the user with the alarm. As a result, an effect of increasing satisfaction of the user with use of a service occurs in operating a health management system utilizing a portable device.

According to an embodiment of the present disclosure, the memory 130 may include at least one type of storage medium among a flash memory type, a hard disk type, a multimedia card micro type, a card type memory (e.g., SD or XD memory, etc.), a Random Access Memory (RAM), a Static Random Access Memory (SRAM), a Read-Only Memory (ROM), an Electrically Erasable Programmable Read-Only Memory (EEPROM), a Programmable Read-Only Memory (PROM), a magnetic memory, a magnetic disk, and an optical disk. The computing device 100 may operate in association with a web storage that performs a storage function of the memory 130 on the Internet. The above description of the memory is merely an example, and the present disclosure is not limited thereto.

The network unit 150 according to an embodiment of the present disclosure may use various wired communication systems such as a Public Switched Telephone Network (PSTN), an x Digital Subscriber Line (xDSL), a Rate Adaptive DSL (RADSL), a Multi Rate DSL (MDSL), a Very High Speed DSL (VDSL), a Universal Asymmetric DSL (UADSL), a High Bit Rate DSL (HDSL), and a Local Area Network (LAN).

In addition, the network unit 150 presented herein may use various wireless communication systems such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

In the present disclosure, the network unit 150 may use any form of wired or wireless communication system.

The techniques described herein may be used in other networks as well as the networks mentioned above.

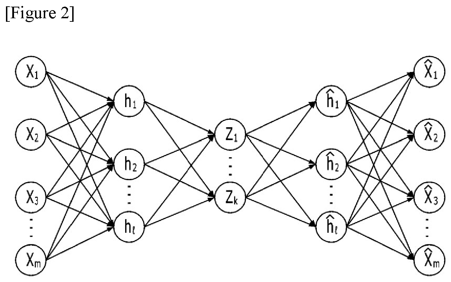

Figure 2 is a schematic diagram illustrating a network function according to an embodiment of the present disclosure.

Throughout this specification, computational model, neural network, network function, and neural network may be used interchangeably. A neural network may generally consist of an interconnected set of computational units, which may be referred to as nodes. Such nodes may also be referred to as neurons. A neural network is configured to include at least one or more nodes. Nodes (or neurons) configuring neural networks may be interconnected by one or more links.

In a neural network, one or more nodes connected through a link may relatively form a relationship of an input node and an output node. The concepts of an input node and an output node are relative, and any node in an output node relationship with respect to one node may be in an input node relationship in a relationship with another node, and vice versa. As described above, the input node to output node relationship may be created around a link. One or more output nodes may be connected to one input node via a link, and vice versa.

In an input node and output node relationship connected via one link, a value of data of the output node may be determined based on data input to the input node. Here, a link interconnecting the input node and the output node may have a weight. The weight may be variable, and may be varied by a user or an algorithm in order for the neural network to perform a desired function. For example, when one or more input nodes are interconnected to one output node by respective links, the output node may determine an output node value based on values input to the input nodes connected to the output node and a weight set for a link corresponding to each of the input nodes.

As described above, a neural network forms a relationship between an input node and an output node within the neural network by one or more nodes being interconnected via one or more links. Characteristics of a neural network may be determined according to the number of nodes and links, correlations between nodes and links, and values of weights assigned to each of the links within the neural network. For example, when there are two neural networks having the same number of nodes and links and different weight values of links, the two neural networks may be recognized as being different from each other.

A neural network may be composed of a set of one or more nodes. A subset of nodes configuring the neural network may configure a layer. Some of the nodes configuring the neural network may configure one layer based on distances from an initial input node. For example, a set of nodes having a distance of n from an initial input node may configure an n layer. The distance from the initial input node may be defined by a minimum number of links that must be traversed to reach a corresponding node from the initial input node. However, such definition of a layer is arbitrary for explanation, and an order of a layer within a neural network may be defined in a method different from that described above. For example, a layer of nodes may be defined by a distance from a final output node.

An initial input node may refer to one or more nodes to which data is directly input without going through a link in a relationship with other nodes among nodes in a neural network. Alternatively, in a relationship between nodes based on a link in a neural network network, it may refer to nodes not having other input nodes connected via a link. Similarly, a final output node may refer to one or more nodes not having an output node in a relationship with other nodes among nodes in a neural network. In addition, a hidden node may refer to nodes configuring a neural network rather than an initial input node and a final output node.

In a neural network according to an embodiment of the present disclosure, the number of nodes in an input layer may be the same as the number of nodes in an output layer, and the neural network may have a form in which the number of nodes decreases and then increases again as it proceeds from the input layer to a hidden layer. Further, in a neural network according to another embodiment of the present disclosure, the number of nodes in an input layer may be smaller than the number of nodes in an output layer, and the neural network may have a form in which the number of nodes decreases as it proceeds from the input layer to a hidden layer. In addition, in a neural network according to another embodiment of the present disclosure, the number of nodes in an input layer may be greater than the number of nodes in an output layer, and the neural network may have a form in which the number of nodes increases as it proceeds from the input layer to a hidden layer. A neural network according to another embodiment of the present disclosure may be a neural network in a combined form of the above-described neural networks.

A deep neural network (DNN) may refer to a neural network including a plurality of hidden layers in addition to an input layer and an output layer. By using a deep neural network, latent structures of data can be identified. That is, latent structures of a photo, text, video, voice, and music (e.g., what objects are in a photo, what contents and emotions of text are, what contents and emotions of voice are, etc.) can be identified. The deep neural network may include a convolutional neural network (CNN), a recurrent neural network (RNN), an auto encoder, Generative Adversarial Networks (GAN), a restricted boltzmann machine (RBM), a deep belief network (DBN), a Q network, a U network, a Siamese network, a Generative Adversarial Network (GAN), and the like. The above description of the deep neural network is merely an example, and the present disclosure is not limited thereto.

In an embodiment of the present disclosure, a network function may include an autoencoder. The autoencoder may be a kind of artificial neural network for outputting output data similar to input data. The autoencoder may include at least one hidden layer, and an odd number of hidden layers may be disposed between an input and output layer. The number of nodes in each layer may be reduced from the number of nodes in an input layer to an intermediate layer called a bottleneck layer (encoding), and may be expanded symmetrically with reduction from the bottleneck layer to an output layer (symmetrical with the input layer). The autoencoder may perform non-linear dimensionality reduction. The number of input layers and output layers may correspond to a dimension after pre-processing of input data. In an autoencoder structure, the number of nodes of a hidden layer included in an encoder may have a structure that decreases as it goes away from an input layer. The number of nodes of a bottleneck layer (a layer located between an encoder and a decoder and having the smallest number of nodes) may be maintained above a certain number (e.g., half or more of an input layer, etc.) since a sufficient amount of information may not be transmitted if it is too small.

A neural network may be trained in at least one method among supervised learning, unsupervised learning, semi-supervised learning, or reinforcement learning. Training of a neural network may be a process of applying knowledge for a neural network to perform a specific action to the neural network.

A neural network may be trained in a direction that minimizes output errors. In the training of a neural network, training data is repeatedly input to the neural network, an error between an output of the neural network and a target for the training data is calculated, and weights of each node of the neural network are updated by backpropagating the error of the neural network from an output layer to an input layer of the neural network in a direction to reduce the error. In the case of supervised learning, training data labeled with a correct answer is used for each training data (i.e., labeled training data), and in the case of unsupervised learning, each training data may not be labeled with a correct answer. That is, for example, training data in the case of supervised learning regarding data classification may be data in which a category is labeled to each training data. The labeled training data is input to a neural network, and an error may be calculated by comparing an output (category) of the neural network and the label of the training data. As another example, an error may be calculated by comparing training data, which is an input, with a neural network output in the case of unsupervised learning regarding data classification. The calculated error is backpropagated in a backward direction (i.e., a direction from an output layer to an input layer) in the neural network, and connection weights of each node of each layer of the neural network may be updated according to the backpropagation. An amount of change in a connection weight of each updated node may be determined according to a learning rate. Calculation of a neural network for input data and backpropagation of an error may configure a learning cycle (epoch). The learning rate may be applied differently depending on the number of repetitions of a learning cycle of a neural network. For example, in an early stage of learning of a neural network, a high learning rate is used so that the neural network quickly secures a certain level of performance to increase efficiency, and in a late stage of learning, a low learning rate is used to increase accuracy.

In training of a neural network, generally, training data may be a subset of actual data (i.e., data to be processed using a trained neural network), and therefore, a training cycle may exist in which an error with respect to the training data decreases but an error with respect to actual data increases. Overfitting is a phenomenon in which an error with respect to actual data increases due to excessive learning on training data as described above. For example, a phenomenon in which a neural network trained on cats by showing a yellow cat fails to recognize a cat other than a yellow cat as a cat may be a kind of overfitting. Overfitting may act as a cause of increasing errors of a machine learning algorithm. In order to prevent such overfitting, various optimization methods may be used. In order to prevent overfitting, methods such as increasing training data, regularization, dropout to deactivate some nodes of a network during a learning process, and utilization of a batch normalization layer may be applied.

Figure 3 is a flowchart illustrating a process of determining a personalized bio signal measurement interval of a user according to an embodiment of the present disclosure.

Referring to Figure 3, the method for determining a personalized bio signal measurement interval of the present disclosure may comprise inputting at least a part of a bio signal of a user into at least one bio signal analysis sub-model (S110), generating comprehensive diagnosis information on a health condition of the user based on an output value generated by the at least one bio signal analysis sub-model (S130), and determining a personalized bio signal measurement interval based on the comprehensive diagnosis information (S150).

In step S110, the processor 110 may input at least a part of a bio signal of a user into at least one bio signal analysis sub-model. In addition, an output value that meets a design purpose of each bio signal analysis sub-model may be generated. For example, when information on a blood pressure is included among bio signals of a user, the processor 110 may input the blood pressure information among the bio signals to a blood pressure analysis sub-model included in a bio signal analysis model, and the blood pressure analysis sub-model may classify bio information of the user as 'normal' or 'abnormal' through the input blood pressure information. Specifically, when a systolic blood pressure is 135 mmHg or more, or when a diastolic blood pressure is 85 mmHg or more, the blood pressure analysis sub-model may classify bio information of the user as 'abnormal', and may classify other cases as 'normal'.

As another example, when information on a blood sugar is included among bio information of a user, the processor 110 may input the blood sugar information among the bio signals to a blood sugar analysis sub-model included in a bio signal analysis model, and the blood sugar analysis sub-model may classify bio information of the user as 'normal' or 'abnormal' through the input blood sugar information. Specifically, when a blood sugar is less than 70 mg/dl or greater than 250 mg/dl, the blood sugar analysis sub-model may classify bio information of the user as 'abnormal', and may classify other cases as 'normal'.

As another example, when information on a body temperature is included among bio information of a user, the processor 110 may input the body temperature information among the bio signals to a body temperature analysis sub-model included in a bio signal analysis model, and the body temperature analysis sub-model may classify bio information of the user as 'normal' or 'abnormal' through the input body temperature information. Specifically, when a body temperature is 35°C or less, the body temperature analysis sub-model may classify bio information of the user as 'abnormal', and may classify other cases as 'normal'.

As another example, when ECG data is included among bio information of a user, the processor 110 may input the ECG data among the bio signals to an ECG analysis sub-model included in a bio signal analysis model, and the ECG analysis sub-model may classify bio information of the user as 'normal' or 'abnormal' through the input ECG.

The ECG analysis sub-model of the present disclosure may include at least one of: a first ECG analysis sub-model estimating a heart age at a current time or predicting heart ages at a plurality of future times by receiving an ECG, and classifying ECG data as 'abnormal' when a difference between the estimated heart age at the current time and an actual age is equal to or greater than a threshold value, when a predicted heart age change at the plurality of future times is equal to or greater than a threshold value, or when a change between a heart age estimated at a past time and a heart age estimated at a current time is equal to or greater than a threshold value; a second ECG analysis sub-model estimating a serum potassium level by receiving an ECG, and classifying ECG data as 'abnormal' when a change in the serum potassium level is equal to or greater than a threshold value; a third ECG analysis sub-model estimating an estimated glomerular filtration rate (eGFR) by receiving an ECG, and classifying ECG data as 'abnormal' when a decrease in the eGFR is equal to or greater than a threshold value; or a fourth ECG analysis sub-model estimating a Left Atrial Size by receiving an ECG, and classifying ECG data as 'abnormal' when the Left Atrial Size is equal to or greater than a threshold value or continuously increases. At this time, each ECG analysis sub-model may be an artificial neural network model.

In step S130, the processor 110 may generate comprehensive diagnosis information on a health condition of the user based on an output value generated by at least one bio signal analysis sub-model.

For example, when at least one of an output value of the first ECG analysis sub-model for estimating a heart age by receiving an ECG, an output value of the second ECG analysis sub-model for estimating a serum potassium level by receiving an ECG, an output value of the third ECG analysis sub-model for estimating an eGFR by receiving an ECG, and an output value of the fourth ECG analysis sub-model for estimating a Left Atrial Size by receiving an ECG is 'abnormal', the processor 110 may generate information such as {'increased risk of heart disease', 'increased risk of stroke', 'kidney function deterioration'} as comprehensive diagnosis information.

In step S150, the processor 110 may DETERMINE A PERSONALIZED BIO SIGNAL MEASUREMENT INTERVAL BASED ON THE COMPREHENSIVE DIAGNOSIS INFORMATION ON THE HEALTH CONDITION OF THE USER. For example, when the comprehensive diagnosis information is {'increased risk of heart disease', 'increased risk of stroke', 'kidney function deterioration'}, the processor 110 may determine a second measurement interval shorter than a first measurement interval, which is an initial measurement interval, as the personalized bio signal measurement interval. As another example, when the comprehensive diagnosis information is {'all bio signals normal'}, the processor 110 may determine a third measurement interval longer than the first measurement interval, which is the initial measurement interval, as the personalized bio signal measurement interval.

In another embodiment of the present disclosure, the bio signal analysis model may be composed of a plurality of bio signal analysis sub-models, and comprehensive diagnosis information on a health condition of a user may be generated by using the plurality of bio signal analysis sub-models at the same time. For example, a plurality of different output values may be generated by a plurality of bio signal analysis sub-models, and the processor 110 may be implemented to generate comprehensive diagnosis information on a health condition of a user based on the different output values.

Figure 4a is a conceptual diagram illustrating a user interface related to a personalized bio signal measurement interval according to an embodiment of the present disclosure.

In the present disclosure, a user interface related to a personalized bio signal measurement interval (hereinafter, a 'first user interface') may be a user interface displayed on a touch screen (not shown) or the like.

The first user interface 410 may include a first area 411 indicating whether a personalized bio signal measurement interval is changed, and a second area 412 displaying a personalized bio signal measurement interval. For example, a text informing a user that a bio signal measurement interval has been changed ('There is a change in a recommended measurement interval') may be displayed in the first area 411, and texts corresponding to a past bio signal measurement interval ('30 days') and a changed bio signal measurement interval ('7 days') may be displayed in the second area 412.

The first user interface 410 may include a third area 413 for receiving a selection input of a user for calling a user interface related to a change in a personalized bio signal measurement interval, and may further include a fourth area 414 for receiving a selection input of a user for removing the first user interface 410.

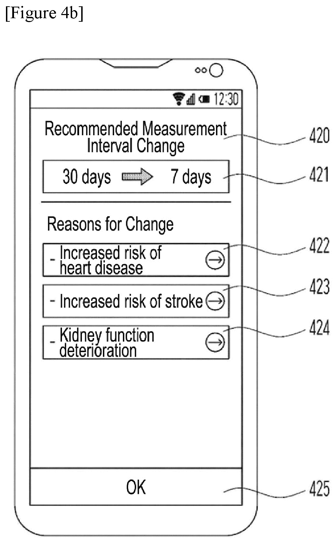

Figure 4b is a conceptual diagram illustrating a user interface related to a change in a personalized bio signal measurement interval according to an embodiment of the present disclosure.

In the present disclosure, a user interface related to a change in a personalized bio signal measurement interval (hereinafter, a 'second user interface') may be a user interface displayed on a touch screen (not shown) or the like. The second user interface 420 may be displayed by a selection input of a user.

The second user interface 420 may include a fifth area 421 for displaying details of a change in a bio signal measurement interval, and sixth areas 422, 423, and 424 for displaying at least one reason for change with respect to the change in the bio signal measurement interval. For example, texts corresponding to a past bio signal measurement interval ('30 days') and a changed bio signal measurement interval ('7 days') may be displayed in the fifth area 421, and texts displaying at least one reason for change ('increased risk of heart disease', 'increased risk of stroke', 'kidney function deterioration') may be displayed in the sixth areas 422, 423, and 424.

The second user interface 420 may further include a seventh area 425 for receiving a selection input of a user to remove the second user interface 420.

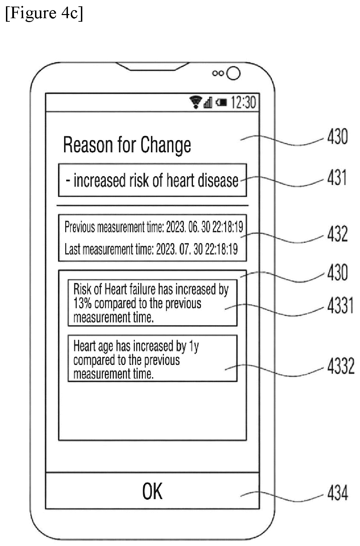

Figure 4c is a conceptual diagram illustrating a user interface related to a reason for change in a personalized bio signal measurement interval according to an embodiment of the present disclosure.

In the present disclosure, a user interface related to a reason for change in a personalized bio signal measurement interval (hereinafter, a 'third user interface') may be a user interface displayed on a touch screen (not shown) or the like. The third user interface 430 may be displayed by a selection input related to a reason for change in a bio signal measurement interval of a user.

The third user interface 430 may include an eighth area 431 for displaying a reason for change in a bio signal measurement interval, a ninth area 432 for displaying information related to a measurement time point of a bio signal, and tenth areas 4331 and 4332 for displaying at least one piece of disease diagnosis information according to a measurement time. At this time, the disease diagnosis information displayed in the tenth areas 4331 and 4332 may include disease diagnosis information output by an artificial neural network model included in a bio signal analysis model of the present disclosure.

For example, a text displaying a reason for change in a bio signal measurement interval ('increased risk of heart disease') may be displayed in the eighth area 431, texts displaying a previous measurement time point and a last measurement time point ('Previous measurement time: 2023. 06. 30 22:18:19', 'Last measurement time: 2023. 07. 30 22:18:19') may be displayed in the ninth area 432, and texts displaying disease diagnosis information ('Risk of Heart failure has increased by 13% compared to the previous measurement time.', 'Heart age has increased by 1y compared to the previous measurement time.') may be displayed in the tenth areas 4331 and 4332.

The third user interface 430 may further include an eleventh area 434 for receiving a selection input of a user for removing the third user interface 430.

Figure 5 is a conceptual diagram illustrating a user interface related to an alarm requesting a bio signal measurement according to an embodiment of the present disclosure.

In the present disclosure, a user interface related to an alarm requesting a bio signal measurement (hereinafter, a 'fourth user interface') may be a user interface displayed on a touch screen (not shown) or the like. The fourth user interface 510 may include a twelfth area 511 displaying information related to a request for a bio signal measurement, a thirteenth area 512 displaying information related to a past bio signal measurement time, a fourteenth area 513 displaying information related to a personalized bio signal measurement interval, and a fifteenth area 514 displaying information related to a reason for change in a personalized bio signal measurement interval.

For example, a text requesting measurement of a bio signal ('Please measure your electrocardiogram') may be displayed in the twelfth area 511, and a text indicating a time elapsed from a past bio signal measurement time point('10 days have elapsed since the last measurement date') may be displayed in the thirteenth area 512.

The fourth user interface 510 may include a sixteenth area 515 for receiving a selection input for ignoring an alarm or a selection input for removing the fourth user interface, and may further include a seventeenth area 516 for receiving a selection input for measuring a bio signal.

Meanwhile, according to an embodiment of the present disclosure, a computer-readable medium storing a data structure is disclosed.

A data structure may refer to organization, management, and storage of data that enable efficient access and modification to data. A data structure may refer to organization of data to solve a specific problem (e.g., retrieving data in the shortest time, storing data, modifying data). A data structure may also be defined as a physical or logical relationship between data elements designed to support a specific data processing function. The logical relationship between data elements may include a connection relationship between user-defined data elements. The physical relationship between data elements may include an actual relationship between data elements physically stored in a computer-readable storage medium (e.g., a permanent storage device). Specifically, a data structure may include a collection of data, relationships between data, functions or instructions applicable to data. Through an effectively designed data structure, a computing device can perform calculations while minimizing use of resources of the computing device. Specifically, a computing device can increase efficiency of calculation, reading, insertion, deletion, comparison, exchange, and retrieval through an effectively designed data structure.

A data structure may be divided into a linear data structure and a non-linear data structure according to a form of the data structure. A linear data structure may be a structure in which only one piece of data is connected after one piece of data. A linear data structure may include a List, a Stack, a Queue, and a Deque. A list may refer to a series of data sets having an internal order. A list may include a Linked List. A linked list may be a data structure in which each piece of data has a pointer and data are connected in a line. In a linked list, a pointer may include connection information with next or previous data. A linked list may be expressed as a singly linked list, a doubly linked list, and a circular linked list according to a form. A stack may be a data arrangement structure having limited access to data. A stack may be a linear data structure capable of processing (e.g., insertion or deletion) data only at one end of a data structure. Data stored in a stack may be a Last In First Out (LIFO) data structure in which data entered later comes out earlier. A queue is a data arrangement structure having limited access to data, and unlike a stack, may be a First In First Out (FIFO) data structure in which data stored later comes out later. A deque may be a data structure capable of processing data at both ends of the data structure.

A non-linear data structure may be a structure in which a plurality of data are connected behind one piece of data. A non-linear data structure may include a Graph data structure. A graph data structure may be defined by a Vertex and an Edge, and an edge may include a line connecting two different vertices. A graph data structure may include a Tree data structure. A tree data structure may be a data structure in which there is one path connecting two different vertices among a plurality of vertices included in a tree. That is, it may be a data structure that does not form a loop in a graph data structure.

Throughout this specification, computational model, neural network, network function, and neural network may be used interchangeably. Hereinafter, it is uniformly described as a neural network. A data structure may include a neural network. And a data structure including a neural network may be stored in a computer-readable medium. A data structure including a neural network may also include data preprocessed for processing by a neural network, data input to a neural network, weights of a neural network, hyperparameters of a neural network, data acquired from a neural network, an activation function associated with each node or layer of a neural network, a loss function for learning of a neural network, and the like. A data structure including a neural network may include any components of the above-disclosed components. That is, a data structure including a neural network may be configured by including all or any combination of data preprocessed for processing by a neural network, data input to a neural network, weights of a neural network, hyperparameters of a neural network, data acquired from a neural network, an activation function associated with each node or layer of a neural network, a loss function for learning of a neural network, and the like. In addition to the above-described configurations, a data structure including a neural network may include any other information determining characteristics of a neural network. Also, a data structure may include all types of data used or generated in a calculation process of a neural network, and is not limited to the above description. A computer-readable medium may include a computer-readable recording medium and/or a computer-readable transmission medium. A neural network may generally consist of an interconnected set of computational units, which may be referred to as nodes. Such nodes may also be referred to as neurons. A neural network is configured to include at least one or more nodes.

A data structure may include data input to a neural network. A data structure including data input to a neural network may be stored in a computer-readable medium. Data input to a neural network may include training data input in a neural network learning process and/or input data input to a trained neural network. Data input to a neural network may include data that has undergone pre-processing and/or data to be subjected to pre-processing. Pre-processing may include a data processing process for inputting data into a neural network. Therefore, a data structure may include data to be subjected to pre-processing and data generated by pre-processing. The above-described data structure is merely an example, and the present disclosure is not limited thereto.

A data structure may include weights of a neural network. (In this specification, weights and parameters may be used in the same meaning.) And a data structure including weights of a neural network may be stored in a computer-readable medium. A neural network may include a plurality of weights. Weights may be variable, and may be varied by a user or an algorithm in order for a neural network to perform a desired function. For example, when one or more input nodes are interconnected to one output node by respective links, an output node may determine a data value output from an output node based on values input to input nodes connected to an output node and weights set for a link corresponding to each input node. The above-described data structure is merely an example, and the present disclosure is not limited thereto.

By way of example and not limitation, weights may include weights varying in a neural network training process and/or weights completely trained for a neural network. Weights varying in a neural network training process may include weights at a start time of a learning cycle and/or weights varying during a learning cycle. Weights completely trained for a neural network may include weights whose learning cycles are completed. Accordingly, a data structure including weights of a neural network may include a data structure including weights varying in a neural network training process and/or weights completely trained for a neural network. Therefore, the above-described weights and/or combinations of respective weights are intended to be included in a data structure including weights of a neural network. The above-described data structure is merely an example, and the present disclosure is not limited thereto.

A data structure including weights of a neural network may undergo a serialization process and then be stored in a computer-readable storage medium (e.g., a memory, a hard disk). Serialization may be a process of converting a data structure into a form capable of being stored in the same or different computing device and reconstructed and used later. A computing device may transmit and receive data over a network by serializing a data structure. A serialized data structure including weights of a neural network may be reconstructed in the same computing device or another computing device through deserialization. A data structure including weights of a neural network is not limited to serialization. Furthermore, a data structure including weights of a neural network may include a data structure (e.g., B-Tree, Trie, m-way search tree, AVL tree, Red-Black Tree in a non-linear data structure) for increasing efficiency of calculation while minimizing use of resources of a computing device. The above description is merely an example, and the present disclosure is not limited thereto.

A data structure may include hyperparameters of a neural network. And a data structure including hyperparameters of a neural network may be stored in a computer-readable medium. A hyperparameter may be a variable varied by a user. A hyperparameter may include, for example, a learning rate, a cost function, the number of learning cycle repetitions, Weight initialization (e.g., setting a range of a weight value to be a target of weight initialization), and the number of Hidden Units (e.g., the number of hidden layers, the number of nodes in a hidden layer). The above-described data structure is merely an example, and the present disclosure is not limited thereto.

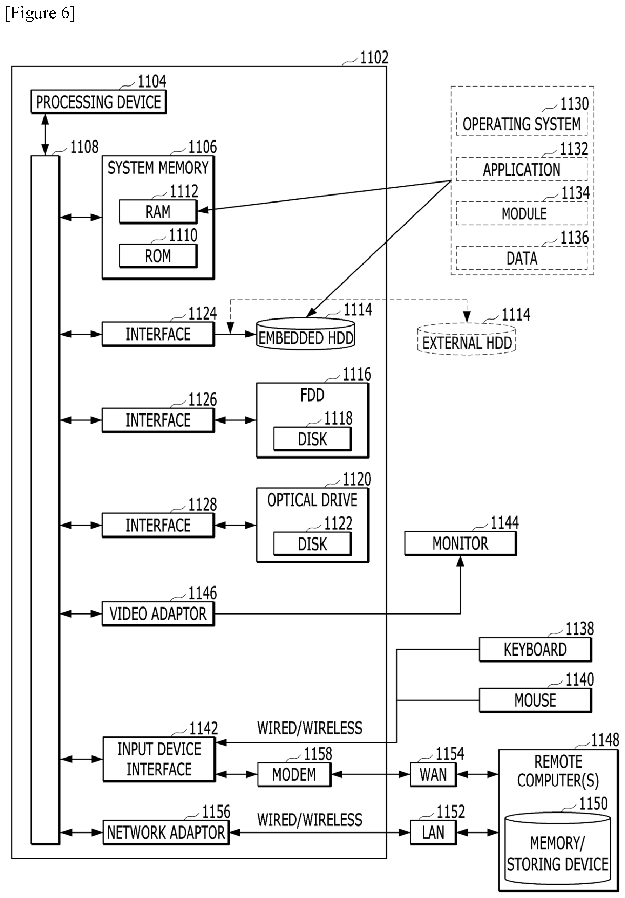

Figure 6 is a brief and general schematic diagram of an exemplary computing environment in which embodiments of the present disclosure may be implemented.

Although the present disclosure has been generally described above as being implementable by a computing device, those skilled in the art will appreciate that the present disclosure may be implemented in combination with computer-executable instructions and/or other program modules capable of being executed on one or more computers and/or as a combination of hardware and software.

Generally, program modules include routines, programs, components, data structures, and the like that perform particular tasks or implement particular abstract data types. In addition, those skilled in the art will appreciate that methods of the present disclosure may be practiced with other computer system configurations, including singleprocessor or multiprocessor computer systems, minicomputers, mainframe computers, as well as personal computers, handheld computing devices, microprocessor-based or programmable consumer electronics, and the like (each of which may be coupled to and operate in conjunction with one or more associated devices).

The described embodiments of the present disclosure may also be practiced in distributed computing environments where certain tasks are performed by remote processing devices linked through a communications network. In a distributed computing environment, program modules may be located in both local and remote memory storage devices.

A computer typically includes a variety of computer-readable media. Any media accessible by a computer can be computer-readable media, and such computer-readable media includes both volatile and nonvolatile media, transitory and non-transitory media, and removable and non-removable media. By way of example, and not limitation, computer-readable media may comprise computer-readable storage media and computer-readable transmission media. Computer-readable storage media include volatile and nonvolatile, transitory and non-transitory, removable and non-removable media implemented in any method or technology for storage of information such as computer-readable instructions, data structures, program modules or other data. Computer-readable storage media include, but are not limited to, RAM, ROM, EEPROM, flash memory or other memory technology, CD-ROM, digital video disk (DVD) or other optical disk storage, magnetic cassettes, magnetic tape, magnetic disk storage or other magnetic storage devices, or any other media which can be accessed by a computer and used to store desired information.

Computer-readable transmission media typically embody computer-readable instructions, data structures, program modules or other data in a modulated data signal such as a carrier wave or other transport mechanism and include any information delivery media. The term modulated data signal means a signal that has one or more of its characteristics set or changed in such a manner as to encode information in the signal. By way of example, and not limitation, computer-readable transmission media include wired media such as a wired network or direct-wired connection, and wireless media such as acoustic, RF, infrared and other wireless media. Combinations of any of the above should also be included within the scope of computer-readable transmission media.

An exemplary environment 1100 for implementing various aspects of the present disclosure including a computer 1102 is shown, and the computer 1102 includes a processing unit 1104, a system memory 1106, and a system bus 1108. The system bus 1108 couples system components including, but not limited to, the system memory 1106 to the processing unit 1104. The processing unit 1104 can be any of various commercially available processors. Dual microprocessors and other multiprocessor architectures may also be employed as the processing unit 1104.

The system bus 1108 may be any of several types of bus structures that may further interconnect to a memory bus, a peripheral bus, and a local bus using any of a variety of commercially available bus architectures. The system memory 1106 includes read-only memory (ROM) 1110 and random access memory (RAM) 1112. A basic input/output system (BIOS) is stored in a non-volatile memory 1110 such as ROM, EPROM, EEPROM, and the BIOS contains basic routines that help to transfer information between elements within the computer 1102, such as during start-up. The RAM 1112 can also include a high-speed RAM such as static RAM for caching data.

The computer 1102 also includes an internal hard disk drive (HDD) 1114 (e.g., EIDE, SATA) - this internal hard disk drive 1114 may also be configured for external use in a suitable chassis (not shown) - a magnetic floppy disk drive (FDD) 1116 (e.g., to read from or write to a removable diskette 1118), and an optical disk drive 1120 (e.g., reading a CD-ROM disk 1122 or, to read from or write to other high capacity optical media such as a DVD). The hard disk drive 1114, magnetic disk drive 1116, and optical disk drive 1120 can be connected to the system bus 1108 by a hard disk drive interface 1124, a magnetic disk drive interface 1126, and an optical drive interface 1128, respectively. The interface 1124 for external drive implementations includes at least one or both of Universal Serial Bus (USB) and IEEE 1394 interface technologies.

These drives and their associated computer-readable media provide nonvolatile storage of data, data structures, computer-executable instructions, and the like. For the computer 1102, the drives and media accommodate storage of any data in a suitable digital format. Although the description of computer-readable media above refers to an HDD, a removable magnetic disk, and a removable optical media such as a CD or DVD, it should be appreciated by those skilled in the art that other types of media which are readable by a computer, such as zip drives, magnetic cassettes, flash memory cards, cartridges, and the like, may also be used in the exemplary operating environment, and further, that any such media may contain computer-executable instructions for performing the methods of the present disclosure.

A number of program modules can be stored in the drives and RAM 1112, including an operating system 1130, one or more application programs 1132, other program modules 1134, and program data 1136. All or portions of the operating system, applications, modules, and/or data can also be cached in the RAM 1112. It will be appreciated that the present disclosure can be implemented with various commercially available operating systems or combinations of operating systems.

A user can enter commands and information into the computer 1102 through one or more wired/wireless input devices, e.g., a keyboard 1138 and a pointing device, such as a mouse 1140. Other input devices (not shown) may include a microphone, an IR remote control, a joystick, a game pad, a stylus pen, a touch screen, or the like. These and other input devices are often connected to the processing unit 1104 through an input device interface 1142 that is coupled to the system bus 1108, but can be connected by other interfaces, such as a parallel port, an IEEE 1394 serial port, a game port, a USB port, an IR interface, and the like.

A monitor 1144 or other type of display device is also connected to the system bus 1108 via an interface, such as a video adapter 1146. In addition to the monitor 1144, a computer typically includes other peripheral output devices (not shown), such as speakers, printers, and the like.

The computer 1102 may operate in a networked environment using logical connections to one or more remote computers, such as a remote computer(s) 1148, via wired and/or wireless communications. The remote computer(s) 1148 can be a workstation, a computing device computer, a router, a personal computer, portable computer, microprocessor-based entertainment appliance, a peer device or other common network node, and typically includes many or all of the elements described relative to the computer 1102, although, for purposes of brevity, only a memory storage device 1150 is illustrated. The logical connections depicted include wired/wireless connectivity to a local area network (LAN) 1152 and/or larger networks, e.g., a wide area network (WAN) 1154. Such LAN and WAN networking environments are commonplace in offices and companies, and facilitate enterprise-wide computer networks, such as intranets, all of which may connect to a global communications network, e.g., the Internet.

When used in a LAN networking environment, the computer 1102 is connected to the local network 1152 through a wired and/or wireless communication network interface or adapter 1156. The adapter 1156 may facilitate wired or wireless communication to the LAN 1152, which also includes a wireless access point disposed thereon for communicating with the wireless adapter 1156. When used in a WAN networking environment, the computer 1102 can include a modem 1158, or is connected to a communications computing device on the WAN 1154, or has other means for establishing communications over the WAN 1154, such as by way of the Internet. The modem 1158, which can be internal or external and a wired or wireless device, is connected to the system bus 1108 via the serial port interface 1142. In a networked environment, program modules depicted relative to the computer 1102, or portions thereof, can be stored in the remote memory/storage device 1150. It will be appreciated that the network connections shown are exemplary and other means of establishing a communications link between the computers can be used.

The computer 1102 operates to communicate with any wireless devices or entities disposed and operating by wireless communication, e.g., a printer, scanner, desktop and/or portable computer, portable data assistant (PDA), communications satellite, any piece of equipment or location associated with a wirelessly detectable tag, and telephone. This includes at least Wi-Fi and Bluetooth wireless technologies. Thus, the communication may be a predefined structure as with a conventional network or simply an ad hoc communication between at least two devices.

Wi-Fi (Wireless Fidelity) allows connection to the Internet from a couch at home, a bed in a hotel room, or a conference room at work, without wires. Wi-Fi is a wireless technology similar to that used in a cell phone that enables such devices, e.g., computers, to send and receive data indoors and out; anywhere within the range of a base station. Wi-Fi networks use radio technologies called IEEE 802.11 (a, b, g, etc.) to provide secure, reliable, fast wireless connectivity. A Wi-Fi network can be used to connect computers to each other, to the Internet, and to wired networks (which use IEEE 802.3 or Ethernet). Wi-Fi networks operate in the unlicensed 2.4 and 5 GHz radio bands, at an 11 Mbps (802.11a) or 54 Mbps (802.11b) data rate, for example, or with products that contain both bands (dual band).

Those skilled in the art will understand that information and signals may be represented using any of a variety of different technologies and techniques. For example, data, instructions, commands, information, signals, bits, symbols, and chips that may be referenced in the above description may be represented by voltages, currents, electromagnetic waves, magnetic fields or particles, optical fields or particles, or any combination thereof.

Those skilled in the art will understand that the various illustrative logical blocks, modules, processors, means, circuits, and algorithm steps described in connection with the embodiments disclosed herein may be implemented by electronic hardware, various forms of program or design code (which is referred to herein as software for convenience), or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Those skilled in the art may implement the described functionality in varying ways for each particular application, but such implementation decisions should not be interpreted as causing a departure from the scope of the present disclosure.

Various embodiments presented herein may be implemented as a method, an apparatus, or an article of manufacture using standard programming and/or engineering techniques. The term article of manufacture includes a computer program, a carrier, or media accessible from any computer-readable storage device. For example, a computer-readable storage medium includes, but is not limited to, a magnetic storage device (e.g., hard disk, floppy disk, magnetic strip, etc.), an optical disk (e.g., CD, DVD, etc.), a smart card, and a flash memory device (e.g., EEPROM, card, stick, key drive, etc.). Also, various storage media presented herein include one or more devices and/or other machine-readable media for storing information.

It is to be understood that a specific order or hierarchical structure of steps in the presented processes is an example of exemplary approaches. It is to be understood that based upon design priorities, a specific order or hierarchical structure of steps in the processes may be rearranged within the scope of the present disclosure. The accompanying method claims present elements of the various steps in a sample order, but are not meant to be limited to the specific order or hierarchical structure presented.

The description of the presented embodiments is provided to enable any person skilled in the art to make or use the present disclosure. Various modifications to these embodiments will be readily apparent to those skilled in the art, and the generic principles defined herein may be applied to other embodiments without departing from the scope of the present disclosure. Thus, the present disclosure is not intended to be limited to the embodiments shown herein, but is to be accorded the widest scope consistent with the principles and novel features presented herein.

### [Mode for Invention]

As described above, related contents have been described in the best mode.

## Claims

1. A method performed by a computing device for determining a bio signal measurement interval, the method comprising:
receiving a bio signal of a user; and
determining a personalized bio signal measurement interval of the user based on inputting the bio signal of the user into a bio signal analysis model,
wherein the bio signal analysis model comprises at least one bio signal analysis sub-model that analyzes a health condition of the user.

2. The method of claim 1, wherein determining the personalized bio signal measurement interval comprises:
inputting at least a part of the bio signal of the user into the at least one bio signal analysis sub-model;
generating comprehensive diagnosis information on the health condition of the user based on an output value generated by the at least one bio signal analysis sub-model; and
determining the personalized bio signal measurement interval based on the comprehensive diagnosis information.

3. The method of claim 2, wherein at least a part of the at least one bio signal analysis sub-model comprises an artificial neural network model, and
wherein the comprehensive diagnosis information is obtained based on disease diagnosis information output by the artificial neural network model.

4. The method of claim 1, further comprising:
displaying a user interface related to the personalized bio signal measurement interval,
wherein the user interface comprises:
an area indicating whether the personalized bio signal measurement interval is changed; and
an area displaying the personalized bio signal measurement interval.

5. The method of claim 1, further comprising:
displaying a user interface related to a change in the personalized bio signal measurement interval in response to a selection input of the user,
wherein the user interface related to the change in the personalized bio signal measurement interval comprises:
an area displaying details of the change in the personalized bio signal measurement interval; and
an area displaying at least one reason for change in the personalized bio signal measurement interval.

6. The method of claim 1, further comprising:
displaying a user interface related to a selected reason for change in response to receiving a selection input related to the reason for change in the bio signal measurement interval of the user,
wherein the user interface related to the selected reason for change comprises:
an area displaying information related to a measurement time point of the bio signal; and
an area displaying disease diagnosis information according to the measurement time point.

7. The method of claim 1, further comprising:
generating alarm information requesting the user to measure the bio signal, based on the personalized bio signal measurement interval.

8. The method of claim 7, wherein generating the alarm information comprises:
displaying a user interface related to the alarm information,
wherein the user interface related to the alarm information comprises:
an area displaying information related to a request for a bio signal measurement;
an area displaying information related to a past bio signal measurement result;
an area displaying information related to the personalized bio signal measurement interval; and
an area displaying information related to a reason for change in the personalized bio signal measurement interval.

9. A computer program stored in a non-transitory computer-readable storage medium, wherein the computer program causes a computing device to perform operations for determining a bio signal measurement interval, the operations comprising:
receiving a bio signal of a user; and
determining a personalized bio signal measurement interval of the user based on inputting the bio signal of the user into a bio signal analysis model,
wherein the bio signal analysis model comprises at least one bio signal analysis sub-model that analyzes a health condition of the user.

10. A computing device comprising:
at least one processor; and
a memory,
wherein the at least one processor is configured to:
receive a bio signal of a user; and
determine a personalized bio signal measurement interval of the user based on inputting the bio signal of the user into a bio signal analysis model,
wherein the bio signal analysis model comprises at least one bio signal analysis sub-model that analyzes a health condition of the user.
